(19) Europäisches Patentamt
European Patent Office
Office européen des brevets

(11) Veröffentlichungsnummer : **0 378 509 B1**

(12) # EUROPÄISCHE PATENTSCHRIFT

(45) Veröffentlichungstag der Patentschrift :
**17.08.94 Patentblatt 94/33**

(51) Int. Cl.$^5$ : **C09B 47/16,** C09B 67/00

(21) Anmeldenummer : **90810003.5**

(22) Anmeldetag : **04.01.90**

(54) **Herstellung von Imidomethylphthalocyanin-Derivaten.**

(30) Priorität : **13.01.89 GB 8900762**

(43) Veröffentlichungstag der Anmeldung :
**18.07.90 Patentblatt 90/29**

(45) Bekanntmachung des Hinweises auf die Patenterteilung :
**17.08.94 Patentblatt 94/33**

(84) Benannte Vertragsstaaten :
**DE DK ES FR GB IT NL**

(56) Entgegenhaltungen :
**DE-C- 852 588**
**US-A- 2 761 868**

(56) Entgegenhaltungen :
**PATENT ABSTRACTS OF JAPAN vol. 6, no. 177 (C-124)(1055) 11. September 1982 & JP-A-57 92 036 (TOYO INK SEIZO K.K. ) 8. Juni 1982**
**H. Beyer, 1978: Lehrbuch der organischen Chemie, 18. Auflage. S. Hirzel Verlag, Stuttgart. S. 483**

(73) Patentinhaber : **CIBA-GEIGY AG**
**Klybeckstrasse 141**
**CH-4002 Basel (CH)**

(72) Erfinder : **MacPherson, Ian Alexander, Dr.**
**12 Hawick Drive**
**Paisley Renfrewshire Scotland (GB)**
Erfinder : **Campbell, Colin Dennis, Dr.**
**c/o Ciba-Geigy Corporation**
**James & Water Street**
**Newport Delaware 19804 (US)**
Erfinder : **Langley, Robert**
**22 Cheviot Drive**
**Newton Mearns**
**Glasgow G77 5AS Scotland (GB)**

EP 0 378 509 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein neues Verfahren zur Herstellung von Imidomethylphthalocyanin-Derivaten, welche z.B. als Anitflockulierungs- und Antikristallisierungszusätze für Beschichtungsmittel und insbesondere als Hitzestabilisatoren für in Ingenieurwerkstoffe (engineering plastics) eingesetzte Pigmente verwendet werden.

Es ist z.B. aus GB-A 695 523 bekannt, Imidomethylphthalocyanine dadurch herzustellen, dass man ein Phthalocyanin mit Formaldehyd und einem cyclischen Imid, unter Dehydratisierungsbedingungen, beispielsweise mittels Schwefelsäure und/oder Oleum, nach folgendem Reaktionsschema

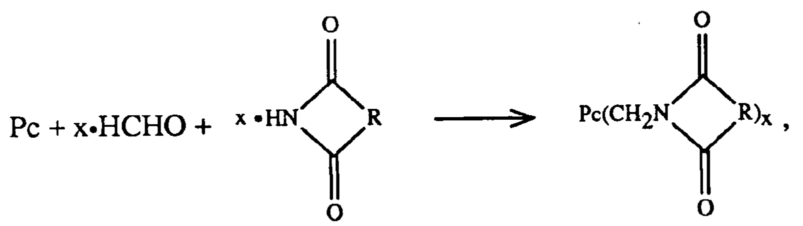

umsetzt, wobei Pc gegebenenfalls metallisiertes Phthalocyanin, R einen zweiwertigen aliphatischen oder aromatischen Rest, wie ein Alkylen- oder Orthophenylen-Rest, und x einen Durchschnittswert von 0,1 bis 4, entsprechend der Zahl von Imidomethylgruppen im Phthalocyanainmolekul, bedeuten.

Ein, gegenüber dem obenerwähnten, etwas abgeändertes Verfahren ist in der US-PS 2 891 964 beschrieben. Dabei werden Formaldehyd und Imid zuerst zu einem Hydroxymethylimid umgesetzt, welches anschliessend mit dem Phthalocyanin unter Dehydratisierungsbedingungen nach dem Reaktionsschema

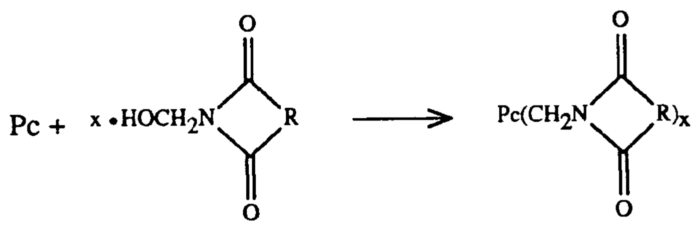

umgesetzt wird, wobei Pc, R und x die oben angegebene Bedeutung haben.

Nach einem weiteren bekannten Verfahren, das beispielsweise in JP-A Sho 55-66584 beschrieben ist, werden Imidomethylphthalocyanin-Derivate durch Umsetzung von chlormethyliertem Phthalocyanin mit einem Metallsalz eines Imids nach dem Reaktionsschema

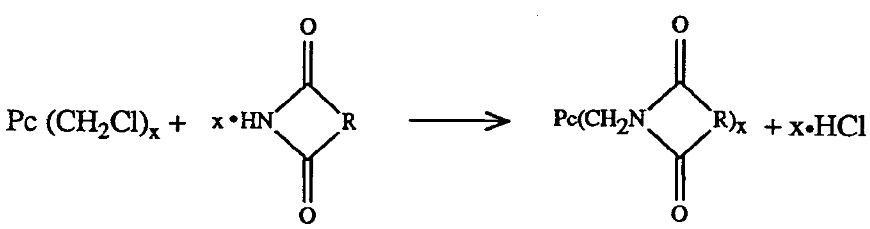

hergestellt, worin Pc, R und x die oben angegebene Bedeutung haben.

Alle diese bekannten Verfahren beruhen auf der Verwendung von Imiden als unentbehrliche Reaktanden.

Es ist nun gefunden worden, dass bei Anwendung eines völlig verschiedenen Reaktionstypus, ausgehend von cyclischen Carbonsäureanhydriden, eine ganze Reihe von Imidomethylphthalocyanin-Derivaten hergestellt werden kann.

Demnach betrifft die vorliegende Erfindung ein Verfahren zur Herstellung von Imidomethylphthalocyanin-Derivaten der Formel

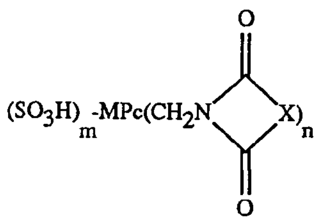

(I),

worin Pc einen unsubstituierten oder durch Chlor oder Brom substituierten Phthalocyaninrest bedeutet, M Wasserstoff oder ein zur Bildung eines Metallphthalocyanins geeignetes Metall ist, m einen Wert zwischen 0,07 und 0,71 und n einen Wert zwischen 0, 1 und 4 bedeuten und X ein Rest ist, das zusammen mit der Gruppe

$$-C(=O)-N-C(=O)- ,$$

ein 5-, 6- oder 7-gliedriges Anhydrid bildet,
dadurch gekennzeichnet, dass ein Aminomethylphthalocyanin der Formel

$$(SO_3H)_m - MPc(CH_2NH_2)_n \quad (II),$$

worin Pc, M, m und n die oben angegebene Bedeutung haben, mit einem 5-, 6- oder 7-gliedrigen cyclischen Anhydrid der Formel

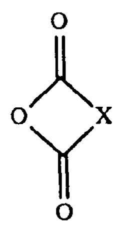

(III),

worin X die oben angegebene Bedeutung hat, umgesetzt wird.

M bedeutet als ein zur Bildung eines Metallphthalocyanins geeignetes Metall z.B. Magnesium, Kobalt, Nickel, Eisen, Zink, Blei, Zinn und insbesondere Kupfer oder Aluminium.

n ist bevorzugt ein Wert zwischen 1,0 und 3,0.

Die Verbindungen der Formel II sind bekannte Verbindungen. Sie werden beispielsweise in der US-PS 2 761 868 und in GB-A 717 137 beschrieben und können dadurch hergestellt werden, dass man das entsprechende Phthalimidomethylphthalocyanin einer alkalischen und danach einer sauren Hydrolyse unterwirft oder, dass man das Phthalimidomethylphthalocyanin einer Hydrazinolyse unterwirft. Die Verbindungen der Formel II werden im erfindungsgemässen Verfahren vorzugsweise als freie Basen eingesetzt. Bevorzugte Verbindung der Formel II ist sulfoniertes Aminomethylkupferphthalocyanin.

Auch bei den Ausgangsprodukten der Formel III handelt es sich um bekannte Verbindungen, wie beispielsweise die folgenden:

a) 5-gliedrige cyclische Anhydride

Bernsteinsäure-, Maleinsäure-, Itaconsäure-, Tetrahydrophthalsäure-, Tetrachlorophthalsäure-, cis-5-Norbornen-endo-2,3-dicarbonsäure-, 3,6-Endoxo- 1,2,3,6-tetrahydrophthalsäure-, Phthalsäure-, 1,2- oder 2,3-Naphthalsäure- oder Chinolinsäure-(Pyridin-2,3-dicarbonsäure-)anhydrid,
jedes davon unsubstituiert oder ein- oder mehrmals durch Halogen, bevorzugt Chlor oder Brom, $C_1$-$C_{20}$-Alkyl, $C_3$-$C_{20}$-Alkenyl, Nitro oder Carboxyl substituiert;

b) 6-gliedrige cyclische Anhydride

Glutarsäure-, 3,3-Tetramethylenglutarsäure-, 1,8-Naphthalsäure-, Perylen-1,12-dicarbonsäure-, Isatosäureanhydrid, jedes davon unsubstituiert oder ein- oder mehrmals durch Halogen, bevorzugt Chlor oder Brom, $C_1$-$C_{20}$-Alkyl, $C_3$-$C_{20}$-Alkenyl, Nitro oder Carboxyl substituiert;

c) 7-gliedrige cyclische Anhydride

Adipinsäure- und Diphensäureanhydri, jedes davon unsubstituiert oder ein- oder mehrmals durch Halogen, bevorzugt Chlor oder Brom, $C_1$-$C_{20}$-Alkyl, $C_3$-$C_{20}$-Alkenyl, Nitro oder Carboxyl substituiert.

Die 5-gliedrigen cyclischen Anhydride sind bevorzugt.

$C_1$-$C_{20}$-Alkyl bedeutet z.B. Methyl, Ethyl, Isopropyl, n-Propyl, n-Butyl, n-Hexyl, n-Octyl, n-Decyl, n-Dodecyl, n-Tetradecyl, n-Octadecyl oder Eicosyl.

$C_3$-$C_{20}$-Alkenyl bedeutet z.B. Propenyl, Butenyl, Pentenyl, Hexenyl, Octenyl, Decenyl, Dodecenyl, Tetradecenyl, Hexadecenyl, Octadecenyl oder Eicosenyl.

Das erfindungsgemässe Verfahren wird zweckmässig bei hohen Temperaturen, bevorzugt zwischen 100 und 300 und insbesondere zwischen 130 und 200°C durchgeführt.

Je nach Temperatur lässt man die Reaktion für eine Zeit, die zwischen 30 Minuten und 12 Stunden, bevorzugt zwischen 3 und 8 Stunden, variieren kann, laufen.

Die Reaktion wird vorzugsweise in einem Lösungsmittel durchgeführt, das einen oder beide Reaktanden löst, aber gegenüber beiden Reaktanden und dem Endprodukt chemisch inert ist. Geeignete Lösungsmittel sind z.B. aromatische Lösungsmittel mit einem Siedepunkt über 100°C, protische Lösungsmittel, wie beispielsweise Alkohole oder Säuren, mit einem Siedepunkt um etwa 100°C, die mit den Anhydriden der Formel III nicht reagieren, und insbesondere aprotische Lösungsmittel mit einem Siedepunkt über 100°C.

Beispiele von bevorzugten aprotischen Lösungsmitteln sind Dimethylsulfoxid, N-Methylpyrrolidon, Tetramethylharnstoff, Ethylenglykol-dimethylether, Dioxan, Hexamethyl-phosphorsäuretriamid Pyridin, Sulfolan, Propylencarbonat, 1 -Formylpiperidin und insbesondere Dimethylformamid.

Die Reaktionen der Aminomethylphthalocyanine der Formel II mit den cyclischen Säureanhydriden der Formel III verlaufen wahrscheinlich nicht vollständig. Unreagiertes Ausgangsmaterial könnte demnach im Endprodukt auftreten. Solches unreagiertes Material hat jedoch keine nachteilige Wirkung auf das Pigment.

Die nachfolgenden Beispiele erläutern die Erfindung. Prozente sind Gewichtsprozente.

Beispiel 1:

a) Herstellung von sulfonierten Aminomethylkupferphthalocyanin

34 g 100%ig sulfoniertes Phthalimidomethylkupferphthalocyanin (enthaltend etwa 2,3 Phthalimidomethylgruppen und 0,07 Sulfosäuregruppen pro Kupferphthalocyaninmolekül) werden als Filterkuchen in 100 ml Wasser bei 90°C aufgeschlämmt. Sobald ein homogener klumpenfreier Teig gebildet ist, werden 15 g 98-100%iges Hydrazinhydrat zugegeben und das Gemisch wird 8 Stunden unter Rühren auf einem auf 130°C erhitzten Oelbad zum Rückfluss gekocht. Das Heizbad wird entfernt, das Gemisch mit Wasser verdünnt und der pH mit Ammoniaklösung (0,88 spezifisches Gewicht) auf 8,0 eingestellt. Das erhaltene Produkt wird abliltriert und mit 1%igem wässrigem Ammoniak aufgeschlämmt, wobei die harten Klumpen zerkleinert werden. Die Aufschlämmung wird filtriert, der Rückstand mit warmem Wasser gewaschen und als Presskuchen mit 22 % Feststoffanteil weiter verwendet. Die Analyse einer getrockneten Probe ergibt einen Gehalt von 2,3 Aminomethylgruppen und von 0,07 Sulfosäuregruppen pro Molekül.

b) Herstellung von sulfoniertem Tetrachlorphthalimidomethylkupferphthalocyanin

100 g sulfoniertes Aminomethylkupferphthalocyanin der Formel

$$(SO_3H)_{0.07}CuPc(CH_2NH_2)_{2.3},$$

erhalten als Presskuchen mit 22 % Feststoffanteil (gemäss Beispiel 1a), werden in 250 g Dimethylformamid aufgeschlämmt und unter gutem Rühren auf einem Oelbad auf 150°C erhitzt. Das gebildete Dimethylformamid/Wasser-Gemisch wird innerhalb von zwei Stunden durch azeotrope Destillation vom Wasser befreit, dann werden 22,9 g Tetrachlorphathalsäureanhydrid zugegeben. Die erhaltene Mischung wird 6 Stunden bei 150°C reagieren gelassen, dann auf 100°C abgekühlt und unter Rühren in 1500 ml kaltes Wasser gegossen. Das ausgefallene feste Produkt wird abfiltriert, mit Wasser gewaschen und getrocknet. Man erhält 40 g sulfoniertes Tetrachlorphthalimidomethylkupferphthalocyanin.

Beispiele 2-12:

Man verfährt wie in Beispiel 1b) und setzt das wie in Beispiel 1a) beschrieben hergestellte sulfonierte Aminomethylkupferphthalocyanin jeweils mit den in Kolonne 2 der nachfolgenden Tabelle aufgeführten Anhydriden um. Man erhält die entsprechenden, in Kolonne 4 der nachfolgenden Tabelle aufgeführten Produkte.

| Beispiel | Anhydrid | Formel | Produkt |
|---|---|---|---|
| 2 | Phthalsäureanhydrid | 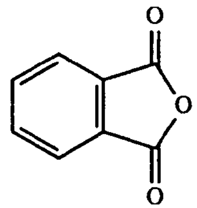 | 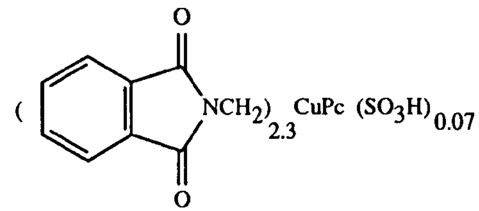 |
| 3 | Trimellithsäureanhydrid | 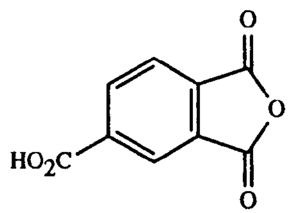 | 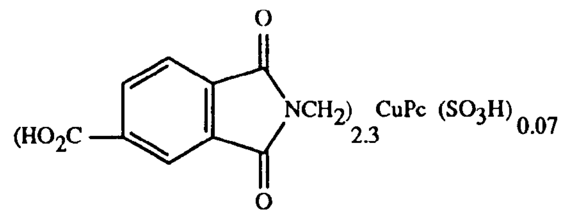 |
| 4 | Tetrabrom-phthalsäureanhydrid | 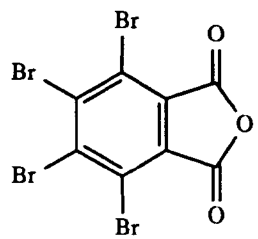 | 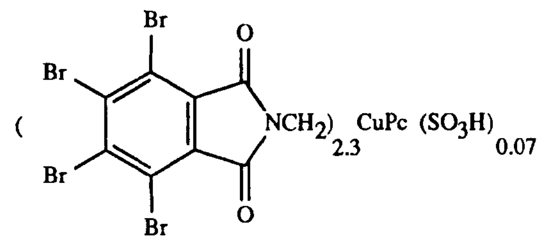 |
| 5 | 1,8-Naphthalsäure-anhydrid | 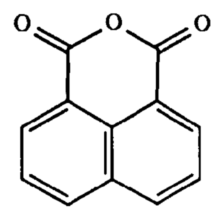 | 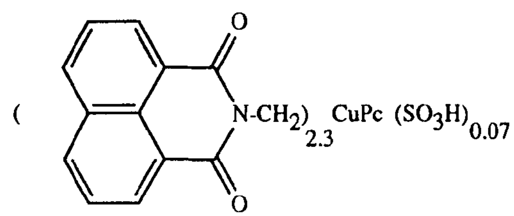 |

| Beispiel | Anhydrid | Formel | Produkt |
|---|---|---|---|
| 6 | Maleinsäureanhydrid | 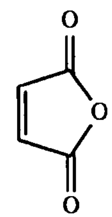 | 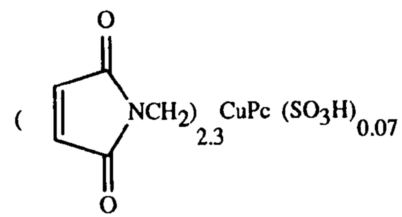 |
| 7 | Citraconsäureanhydrid | 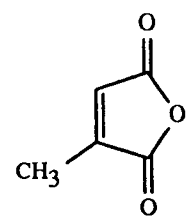 | 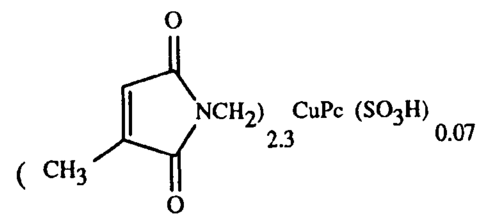 |
| 8 | Dimethylmaleinsäure-anhydrid | 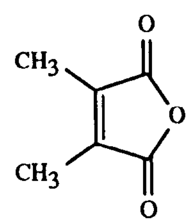 | 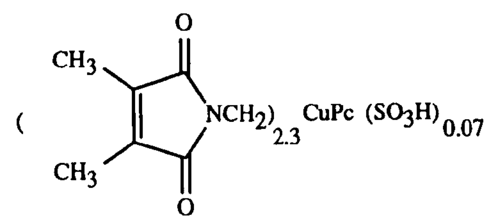 |
| 9 | Dichlormaleinsäure-anhydrid | 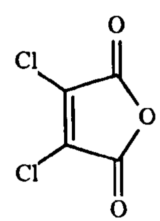 | 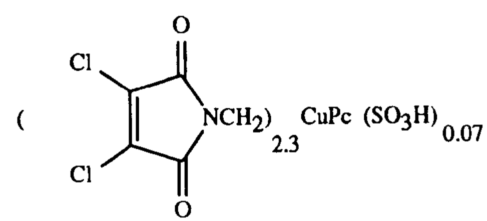 |

| Beispiel | Anhydrid | Formel | Produkt |
|---|---|---|---|
| 10 | Bernsteinsäureanhydrid | 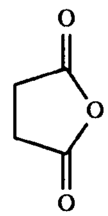 | 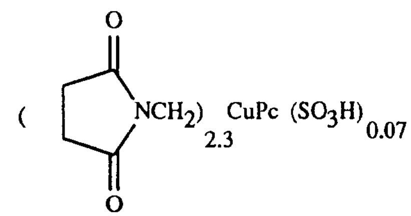 |
| 11 | Oleyl-Bernsteinsäure-anhydrid | 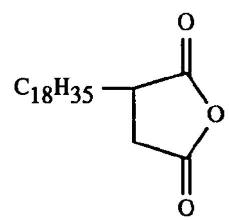 | 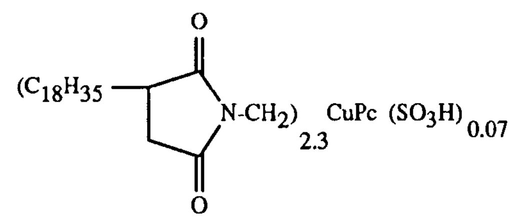 |
| 12 | Chlorendianhydrid | 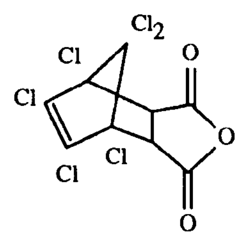 | 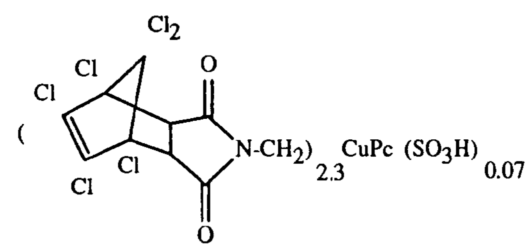 |

Beispiel 13:

a) Herstellung von sulfonierten Aminomethylkupferphthalocyanin

Sulfoniertes Phthalimidomethylkupferphthalocyanin enthaltend etwa 1,4-Phthalimidomethyl- und 0,71 Sulfogrupppen pro Kupferphthalocyaninmolekül wird wie in Beispiel 1a) beschrieben mit Hydrazinhydrat umgesetzt, wobei ein sulfoniertes Aminomethylkupferphthalocyanin der Formel $(SO_3H)_{0,71}CuPc(CH_2NH_2)_{1,4}$ erhalten wird.

b) Herstellung von sulfoniertem Phthalimidomethylkupferphthalocyanin

85 g des gemäss Beispiel 13a) erhaltenen Materials werden als Presskuchen mit 9,5 % Feststoffanteil in 250 g Dimethylformamid aufgeschlämmt und, unter kräftigem Rühren, auf 150°C erhitzt. Das gebildete Dimethylformamid/Wasser-Gemisch wird zur Entfernung des Wassers azeotrop destilliert, dann werden 4,12 g Phthalsäureanhydrid zugegeben. Die erhaltene Mischung wird 6 Stunden bei 150°C reagieren gelassen, dann auf 100°C abgekühlt und unter gutem Rühren in 1000 ml kaltes Wasser gegossen. Das ausgefallene feste Produkt wird abfiltriert, gut mit Wasser gewaschen und getrocknet. Man erhält 9,2 g sulfoniertes Phthalimidomethylkupferphthalocyanin enthaltend 0,71 Sulfosäure- und 1,4-Phthalimidomethylgruppen pro Kupferphthalocyaninmolekül.

Beispiel 14:

a) Herstellung von sulfoniertem Aminomethylaluminiumphthalocyanin

Sulfoniertes Aminomethylaluminiumphthalocyanin enthaltend etwa 2,5 Aminomethyl- und 0,7 Sulfosäuregruppen wird in Analogie zu dem unter Beispiel 1a) beschriebenen Verfahren hergestellt.

b) Herstellung von sulfoniertem Tetrachlorphthalimidomethylaluminiumphthalocyanin

100 g des gemäss Beispiel 14a) erhaltenen sulfonierten Aminomethylaluminiumphthalocyanins werden als Presskuchen mit 18 % Feststoffanteil in 250 g Dimethylformamid aufgeschlämmt. Die Aufschlämmung wird unter Rühren auf 150°C erhitzt und das Wasser des gebildeten Dimethylformamid/Wasser-Gemisches wird azeotrop abdestilliert, bevor 24,3 g Tetrachlorphthalsäureanhydrid zugegeben werden. Die erhaltene Mischung wird 6 Stunden bei 150°C reagieren gelassen, dann auf 100°C abgekühlt und unter gutem Rühren in 1000 ml kaltes Wasser gegossen. Das Produkt wird abfiltriert, mit Wasser gewaschen und getrocknet. Man erhält 27,5 g sulfoniertes Tetrachlorphthalimidomethylaluminiumphthalocyanin.

Beispiel 15:

Herstellung von sulfiniertem Diphenimidomethylkupferphthalocyanin

49 g sulfoniertes Aminomethylkupferphthalocyanin, erhalten nach der Vorschrift von Beispiel 1a), werden als Presskuchen mit 20,4 % Feststoffanteil in 250 g Dimethylsulfoxid aufgeschlämmt und unter gutem Rühren erhitzt, bis das Wasser vollständig entfernt ist. Die Mischung wird auf 80°C abgekühlt, dann werden 8,5 g Diphensäureanhydrid portionenweise unter Rühren der Aufschlämmung zugegeben. Die Temperatur wird auf 160°C erhöht, die Mischung wird weitere 6 Stunden bei dieser Temperatur gerührt, auf 100°C abgekühlt und anschliessend unter gutem Rühren in 1000 ml kaltes Wasser gegossen. Die Suspension wird filtriert, der Rückstand mit Wasser gewaschen und getrocknet. Man erhält ein sulfoniertes Diphenimidomethylkupferphthalocyanin enthaltend 0,07 Sulfosäure- und 2,3 Diphenimidomethylgruppen pro Kupferphthalocyaninmolekül.

Beispiel 16:

29,3 g Kupferphthalocyanin, 55,0 g wasserfreies Calciumchlorid und 5 g Natriumacetat krist. werden 12 Stunden in Gegenwart von 12 mm-Stahlkugeln in einer Vibrationsmühle gemahlen und dann in 280 g eines Isopropanol/Wasser-Gemisches (83:17) enthaltend 10 g einer 16,6%igen alkalischen Kolophoniumlösung aufgeschlämmt. Die Mischung wird eine Stunde unter Rühren am Rückfluss gekocht, dann werden 160 ml heisses Wasser zugegeben. Das Isopropanol wird azeotrop abdestilliert und 68 ml kaltes Wasser werden dem Rückstand zugefügt.

1,6 g des Produkts von Beispiel 1b) (100%ig) werden in Wasser aufgeschlämmt und zur Kupferphthalocyaninaufschlämmung gegeben. Nach 30 Minuten Rühren werden 16,6 g 36%ige Salzsäure zugegeben und die Reaktionsmischung wird eine Stunde bei 50-60°C gerührt. Das Pigment wird abliltriert, mit kaltem Wasser gewaschen, bis das Filtrat chloridfrei ist, dann bei 60°C getrocknet und durch ein Sieb mit einer Maschenweite von 150 µm gesiebt.

In eine Zweiwalzenmühle, deren Walzen bei Walzenabstand von 0,3 mm auf 150 bzw. 110°C geheizt werden, werden 100 g Polyethylen hoher Dichte eingeführt. Das Polymere wird eine Minute im Walzenstuhl gemahlen, dann wird 0,1 g des wie oben beschrieben hergestellten Pigmentes innerhalb 30 Sekunden auf das Polymere gestreut. Nach dem Mahlen während 8 Minuten wird der Walzenabstand auf 1,5 mm eingestellt und die Folie wird abgezogen, auf Raumtemperatur abkühlen gelassen und abgeschnitzelt. Das abgeschnitzelte Material wird in einer Spritzgussmaschine bei 200°C zu intensiv blauen Formstücken verspritzt. Das Verfahren wird mehrmals wiederholt, wobei die Temperatur der Spritzgussmaschine jedes Mal um 20°C bis 320°C erhöht wird (5 Minuten Verweilzeit bei jeder Temperatur).

Die erhaltenen blauen Formstücke zeigen eine unveränderte Nuance bis zu einer erheblich höheren Temperatur, als diejenigen, bei denen kein sulfoniertes Tetrachlorophthalimidomethylkupferphathalocyanin zugegeben wird.

Beispiel 17:

Man verfährt wie in Beispiel 13b) und setzt 22 g (Trockengewicht) des wie in Beispiel 1a) beschrieben hergestellten sulfonierten Aminomethylkupferphthalocyanins mit 22,9 g Phthalsäureanhydrid in Dimethylformamid zu sulfoniertem Phthalimidomethylkupferphthalocyanin um. Die Reaktion wird während 8 Stunden bei 100°C durchgeführt.

Beispiel 18:

Man verfährt wie in Beispiel 13b) und setzt 22 g (Trockengewicht) des wie in Beispiel 1a) beschrieben hergestellten sulfonierten Aminomethylkupferphthalocyanins mit 11,9 g Phthalsäureanhydrid in N-Methylpyrrolidon zu sulfoniertem Phthalimidomethylkupferphthalocyanin um. Die Reaktion wird während 3 Stunden bei 200°C durchgeführt.

**Patentansprüche**

1. Verfahren zur Herstellung von Imidomethylphthalocyanin-Derivaten der Formel

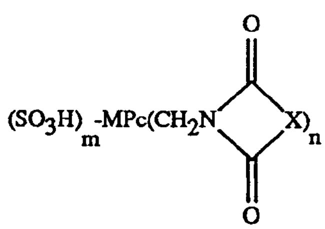

(I),

worin Pc einen unsubstituierten oder durch Chlor oder Brom substituierten Phthalocyaninrest bedeutet, M Wasserstoff oder ein zur Bildung eines Metallphthalocyanins geeignetes Metall ist, m einen Wert zwischen 0,07 und 0,71 und n einen Wert zwischen 0, 1 und 4 bedeuten und X ein Rest ist, das zusammen mit der Gruppe

$$-C(=O)-\underset{|}{N}-C(=O)-$$

ein 5-, 6- oder 7-gliedriges Anhydrid bildet,
dadurch gekennzeichnet, dass ein Aminomethylphthalocyanin der Formel

$$(SO_3H)_m - MPc(CH_2NH_2)_n \quad (II),$$

worin Pc, M, m und n die oben angegebene Bedeutung haben, mit einem 5-, 6- oder 7-gliedrigen cyclischen Anhydrid der Formel

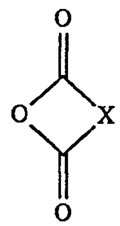

(III),

worin X die oben angegebene Bedeutung hat, umgesetzt wird.

2. Verfahren gemäss Anspruch 1, dadurch gekennzeichnet, dass M Magnesium, Kobalt, Nickel, Eisen, Zink, Blei, Zinn, Kupfer oder Aluminium ist.

3. Verfahren gemäss Anspruch 1, dadurch gekennzeichnet, dass M Kupfer oder Aluminium ist.

4. Verfahren gemäss Anspruch 1, dadurch gekennzeichnet, dass n einen Wert zwischen 1,0 und 3,0 bedeutet.

5. Verfahren gemäss Anspruch 1, dadurch gekennzeichnet, dass die Verbindung der Formel II sulfoniertes Aminomethylkupferphthalocyanin ist.

6. Verfahren gemäss Anspruch 1, dadurch gekennzeichnet, dass die Verbindung der Formel III Bernsteinsäure-, Maleinsäure-, Itaconsäure-, Tetrahydrophthalsäure-, Tetrachlorophthalsäure-, cis-5-Norbornen-endo-2,3-dicarbonsäure-, 3,6-Endoxo-1,2,3,6-tetrahydrophthalsäure-, Phthalsäure-, 1,2- oder 2,3-Naphthalsäure- oder Chinolinsäure-(Pyridin-2,3-dicarbonsäure-)anhydrid, jedes davon unsubstituiert oder ein- oder mehrmals durch Halogen, $C_1$-$C_{20}$-Alkyl, $C_3$-$C_{20}$-Alkenyl, Nitro oder Carboxyl substituiert, ist.

7. Verfahren gemäss Anspruch 1, dadurch gekennzeichnet, dass es bei Temperaturen zwischen 100 und 300°C durchgeführt wird.

8. Verfahren gemäss Anspruch 1, dadurch gekennzeichnet, dass es in Gegenwart eines Lösungsmittels durchgeführt wird, das einen oder beide Reaktanden löst, aber gegenüber beiden Reaktanden und dem Endprodukt chemisch inert ist.

9. Verfahren gemäss Anspruch 1, dadurch gekennzeichnet, dass das Lösungsmittel ein aprotisches Lösungsmittel ist.

10. Verfahren gemäss Anspruch 8, dadurch gekennzeichnet, dass das Lösungsmittel Dimethylformamid ist.

## Claims

1. A process for the production of an imidomethyl phthalocyanine derivative of the formula

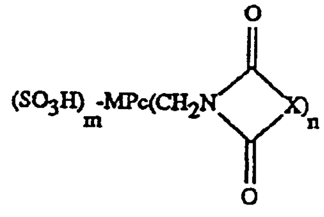

(I),

in which Pc is a phthalocyanine radical which is unsubstituted or substituted by chlorine or bromine; M is hydrogen, or a metal capable of forming a metal phthalocyanine; m is a value between 0.07 and 0.71 and n is a value between 0.1 and 4 and X is a radical which , in combination with the group

$$-C(=O)-N-C(=O)- ,$$

forms a 5-, 6- or 7-membered anhydride, which process comprises reacting an aminomethyl phthalocyanine of the formula

$$(SO_3H)_m - MPc(CH_2NH_2)_n \quad (II),$$

in which Pc, M, m and n are as defined above, with a 5-, 6- or 7-membered cyclic anhydride of the formula

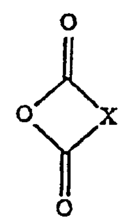

(III),

in which X is as defined above.

2. A process according to claim 1, wherein M is magnesium, cobalt, nickel, iron, zinc, lead, tin, copper or aluminium.

3. A process according to claim 1, wherein M is copper or aluminium.

4. A process according to claim 1, wherein n is a value between 1.0 and 3.0.

5. A process according to claim 1, wherein the compound of the formula II is sulfonated aminomethyl copper phthalocyanine.

6. A process according to claim 1, wherein the compound of the formula III is succinic anhydride, maleic anhydride, itaconic anhydride, tetrahydrophthalic anhydride, tetrachlorophthalic anhydride, cis-5-norbornene-endo-2,3-dicarboxylic anhydride; 3,6-endoxo-1,2,3,6-tetrahydrophthalic anhydride, phthalic anhydride, 1,2- or 2,3-naphthalene anhydride or quinolinic anhydride (pyridine-2,3-dicarboxylic anhydride) each unsubstituted or monosubstituted or polysubstituted by halogen, $C_1$-$C_{20}$alkyl, $C_3$-$C_{20}$alkenyl, nitro or carboxyl.

7. A process according to claim 1, wherein the process is performed at a temperature between 100 and 300°C.

8. A process according to claim 1, wherein the process is performed in the presence of a solvent which dissolves one or both of the reactants while being chemically inert towards both reactants and towards the end product.

9. A process according to claim 1, wherein the solvent is an aprotic solvent.

10. A process according to claim 8, wherein the solvent is dimethylformamide.

## Revendications

1. Procédé de préparation de dérivés de l'imidométhylphtalocyanine répondant à la formule

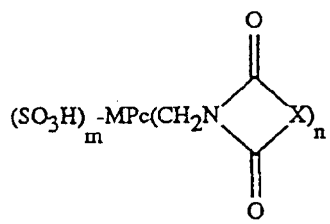

(I),

dans laquelle Pc désigne un radical phtalocyanine non substitué ou substitué par des atomes de chlore ou de brome, M est un atome d'hydrogène ou un métal approprié à la formation d'une phtalocyanine métallique, m a une valeur comprise entre 0,07 et 0,71 et n a une valeur comprise entre 0, 1 et 4, X est un radical qui forme avec le groupe -C(=O)-N-C(=O)- un anhydride à 5, 6 ou 7 maillons, caractérisé en ce qu'on fait réagir une aminométhylphtalocyanine répondant à la formule

$$(SO_3H)_m - MPc(CH_2NH_2)_n \quad (II)$$

dans laquelle Pc, M, m et n ont la signification indiquée ci-dessus, avec un anhydride cyclique à 5, 6 ou 7 maillons répondant à la formule

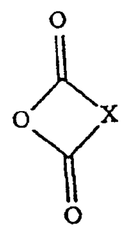

(III),

dans laquelle X a la signification indiquée ci-dessus.

2. Procédé selon la revendication 1, caractérisé en ce que M est le magnésium, le cobalt, le nickel, le fer, le zinc, le plomb, l'étain, le cuivre ou l'aluminium.

3. Procédé selon la revendication 1, caractérisé en ce que M est le cuivre ou l'aluminium.

4. Procédé selon la revendication 1, caractérisé en ce que n a une valeur comprise entre 1,0 et 3,0.

5. Procédé selon la revendication 1, caractérisé en ce que le composé répondant à la formule II est une aminométhylphtalocyanine de cuivre sulfonée.

6. Procédé selon la revendication 1, caractérisé en ce que le composé répondant à la formule III est l'anhydride succinique, maléique, itaconique, tétrahydrophtalique, tétrachlorophtalique, cis-5-norbornène-endo-2,3-dicarboxylique, 3,6-endoxo-1,2,3,6-tétrahydrophtalique, phtalique, 1,2- ou 2,3-naphtalique ou quinolinique-(pyridine-2,3-diarboxylique), chacun de ceux-ci étant non substitué ou substitué une ou plusieurs fois par des atomes d'halogène, des groupes alkyle en $C_1$-$C_{20}$, alcényle en $C_3$-$C_{20}$, nitro ou carboxyle.

7. Procédé selon la revendication 1, caractérisé en ce qu'on opère à des températures comprises entre 100 et 300 °C.

8. Procédé selon la revendication 1, caractérisé en ce qu'on opère en présence d'un solvant qui dissout un des réactifs ou les deux, mais qui est chimiquement inerte vis-à-vis des deux réactifs et du produit final.

9. Procédé selon la revendication 1, caractérisé en ce que le solvant est un solvant aprotique.

10. Procédé selon la revendication 8, caractérisé en ce que le solvant est le diméthylformamide.